(19) **Europäisches Patentamt / European Patent Office / Office européen des brevets**

(11) **EP 4 559 459 A1**

(12) **EUROPEAN PATENT APPLICATION**
published in accordance with Art. 153(4) EPC

(43) Date of publication:
**28.05.2025 Bulletin 2025/22**

(21) Application number: **23842224.0**

(22) Date of filing: **14.07.2023**

(51) International Patent Classification (IPC):
**A61K 9/72** *(2006.01)*   **A61K 9/08** *(2006.01)*
**A61K 31/198** *(2006.01)*   **A61M 11/00** *(2006.01)*
**A61M 15/00** *(2006.01)*   **A61P 11/00** *(2006.01)*
**A61P 11/06** *(2006.01)*   **A61P 11/10** *(2006.01)*
**A61P 31/06** *(2006.01)*

(52) Cooperative Patent Classification (CPC):
**Y02A 50/30**

(86) International application number:
**PCT/CN2023/107448**

(87) International publication number:
**WO 2024/017161 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.07.2022   CN 202210842971**

(71) Applicant: **Beijing Increase Innovative Drug Co., Ltd.**
**Beijing 102200 (CN)**

(72) Inventors:
• **ZHANG, Baoxian**
  **Beijing 102200 (CN)**
• **HU, Jie**
  **Beijing 102200 (CN)**
• **WEI, Huanli**
  **Beijing 102200 (CN)**
• **LI, Cui**
  **Beijing 102200 (CN)**
• **GONG, Li**
  **Beijing 102200 (CN)**

(74) Representative: **ABG Intellectual Property Law, S.L.**
**Avenida de Burgos, 16D**
**Edificio Euromor**
**28036 Madrid (ES)**

(54) **FUDOSTEINE NEBULIZATION INHALATION SOLUTION COMPOSITION, DRUG ASSEMBLY, AND USE THEREOF**

(57)   A fudosteine nebulization inhalation solution composition and use thereof. The fudosteine nebulization inhalation solution consists of fudosteine, a pH-adjusting agent and water. The droplet particle size of the composition is as follows: the $D_{90}$ particle size is 7.0-10.0 $\mu$m, the $D_{50}$ particle size is 2.8-4.5 $\mu$m, and the $D_{10}$ particle size is 0.5-1.5 $\mu$m; and the percent deposition of the particles with the aerodynamic particle size of 1.4-5.4 $\mu$m in the fudosteine inhalation solution is not less than 50%. In addition, further provided is a drug assembly for use in conjunction with the nebulization inhalation solution, comprising the inhalation composition, and a nebulizer and a nebulizing cup with specific parameters. The fudosteine nebulization inhalation solution composition and the assembly thereof have the advantages of a small effective dose, acting fast, high drug delivery efficiency, and the like, and are particularly effective in treating tracheal and main bronchial diseases.

drug distribution results under different driver flow rates

- 2L/min
- 4L/min
- 6L/min
- 8L/min

percent deposition/%

particle size range 2.1μm~5.4μm   particle size range 1.4μm~2.1μm

FIG. 1

## Description

This application claims the priority of Chinese Patent Application No. 202210842971.5, filed with the China National Intellectual Property Administration on July 18, 2022, and titled with "FUDOSTEINE NEBULIZATION INHALATION SOLUTION COMPOSITION, DRUG ASSEMBLY, AND USE THEREOF", which is incorporated herein by reference in its entirety.

## FIELD

The present disclosure relates to the technical field of nebulization inhalation, and specifically relates to a fudosteine nebulised inhalation solution composition, a drug assembly and use thereof.

## BACKGROUND

Nebulised inhalation solution composition means a preparation in which drugs are administered by a special device and exert systemic or local effects through deep respiratory tract, cavity and mucosa, etc. Compared with the traditional way of administration, nebulization administration has the advantages of rapidity, high efficiency and good patient compliance, which avoids the first-pass effect of the liver and reduces the possible adverse reactions caused by systemic administration. It is currently recognized as the best way to treat respiratory diseases such as asthma and chronic pulmonary obstructive disease (COPD) in the world. In recent years, with the increasing severity of air pollution and the continued deepening of aging of the population, the prevalence of respiratory diseases has been rising, and the nebulization inhalation administration has become a kind of administration method that has attracted much attention.

As a specific dosage form for administration, nebulised inhalation solution is converted into aerosol by a specific device, so that the inhaled dose is deposited in the respiratory system at a certain rate and with a suitable particle size, and especially the particle size, which is widely studied by researchers as a preparation factor that mainly affects the nebulization administration.

Fudosteine was first marketed in Japan in 2001 and produced by SSP and Mitsubishi Welfide. It is a cysteine derivative drug, which can change the composition and rheological properties of secretions, reduce sputum viscosity and improve suppressed respiratory function. In addition, fudosteine can also improve the secretion of serous trachea, thus inhibiting tracheal inflammation.

At present, fudosteine-related drugs that have been marketed domestically and abroad are all oral dosage forms (including oral solutions, tablets, capsules and granules). CN113975262A discloses a pharmaceutical composition containing fudosteine and a preparation method thereof. The pharmaceutical composition comprises fudosteine, a thickener, a flavoring agent, a pH-adjusting agent, a colorant, a fragrance and a solvent. The pharmaceutical composition comprises a thickener and a pH-adjusting agent at the same time, and the thickener and the pH-adjusting agent cooperate with each other to jointly maintain the excellent stability of the drug composition. The pharmaceutical composition is an oral preparation, and for the purpose of improving the taste, adjuvants such as colorant, fragrance are added. CN112057418A discloses a fudosteine oral solution and a preparation method thereof. The fudosteine oral solution comprises fudosteine, a metal ion compound, an acid-base regulator and a solvent, wherein fudosteine and the metal ion form a chelate through chelation, which solves the problem that the impurity content of isomers in the fudosteine oral solution increases rapidly with time. However, on the one hand, the oral dosage form is difficult to be applied for patients suffering from gastrointestinal diseases and unable to swallow and coma; on the other hand, fudosteine can damage the gastric mucosal barrier, which easily leads to adverse reactions such as rash, nausea, indigestion and itching, hypoesthesia, abdominal discomfort and diarrhea.

China patent CN108078964A discloses a fudosteine inhalation composition, and relates to a fudosteine dry powder inhalation preparation and a preparation method thereof. The purpose is to provide a fudosteine dry powder inhalation agent with low dosage, fast effect and direct drug attack on target lesions and preparation method thereof, aiming at the defects of the commercially available fudosteine dosage forms, which has large dosage and insufficient systemic action. However, the dry powder inhalation device is relatively expensive, which increases the cost of medication and increases the burden on patients. At the same time, the internal flow resistance of the device affects the delivery and distribution of drugs, thus affecting the efficacy.

US20200316003A1 first discloses a solution preparation for fudosteine nebulization inhalation, which prepares a single dose solution preparation for fudosteine nebulization inhalation, comprising fudosteine or salt and/or hydrate thereof, a metal complexing agent EDTA, a pH-adjusting agent and water for injection. It has the characteristics of high efficiency, low toxicity, good stability and high safety. However, there are still problems to be solved when using nebulizer for administration, such as wide droplet size distribution, large geometric standard deviation range, large residual liquid volume, prolonged treatment time and reduced compliance.

The drug action site of the existing drugs after being administered by an air compression nebulizer is generally the lung, while fudosteine is mainly used as a mucolytic agent and can act well on goblet cells, and goblet cells are mainly distributed in trachea and main bronchus. Therefore, in order to achieve better therapeutic effect of fudosteine, it is necessary to solve the problem of fudosteine administration site, specifically, to enable fudosteine to be mainly deposited in trachea and main bronchus.

Therefore, how to develop a fudosteine nebulised inhalation solution composition and a drug assembly to solve the above-mentioned problems is an urgent problem to be solved at present.

## SUMMARY

In order to solve the existing technical problem of the drug action site of fudosteine after nebulization inhalation, the present disclosure provides a fudosteine nebulised inhalation solution composition with uniform particle size distribution, high utilization rate of drug solution, short treatment time and remarkable efficacy. The present disclosure also provides a drug assembly of the fudosteine inhalation solution. By combining the fudosteine inhalation solution with the compression nebulizer, fudosteine nebulization particles can directly act on trachea and main bronchus, thus achieving the most ideal therapeutic effect, significantly improving the safety and effectiveness of medication for patients, and solving the existing technical problem that fudosteine can only be mostly deposited in the lungs.

In order to achieve the above-mentioned object of the present disclosure, the technical solutions according to the present disclosure are as follows:

A fudosteine nebulised inhalation solution composition is provided according to the first object of the present disclosure, which comprises fudosteine or a pharmaceutically acceptable salt thereof as an active ingredient, a pH-adjusting agent and water for injection.

Preferably, the composition has a pH of 2.5-5.0.

Preferably, the composition has a pH of 3.5-4.0.

Further preferably, the composition has a pH of 3.7-3.9.

The physical properties of the nebulised inhalation solution preparation determine the distribution of aerodynamic diameter of nebulization particles, and the aerodynamic diameter of nebulization particles and the various parameters of nebulizing device have important influence on the administration site. The inventor studied the main proliferation sites of goblet cells in the respiratory tract in the efficacy research test, and found that goblet cells were scattered in the trachea and main bronchus of the respiratory tract, indicating that goblet cells were mainly located in trachea and main bronchus. As an expectorant and respiratory mucolytic agent, fudosteine can reduce the viscosity of sputum by inhibiting the excessive formation of goblet cells secreting sticky sputum in trachea, which is easy to cough up, thus playing an expectorant role. Therefore, fudosteine has a good expectorant effect on goblet cells. Based on this, the expectorant effect is better when the administration site of fudosteine is trachea and main bronchus. According to the drug assembly of the fudosteine inhalation solution in the present disclosure, the fudosteine inhalation solution is used in conjunction with a compression nebulizer, so that nebulization particles can directly act on trachea and main bronchus, thereby achieving the most ideal treatment effect.

Preferably, the inhalation solution has following droplet particle size during nebulization inhalation: D90 particle size of 7.0-10.0 $\mu$m, D50 particle size of 2.8-4.5 $\mu$m, and D10 particle size of 0.5-1.5 $\mu$m; the percent deposition of the particles with aerodynamic particle size of 1.4-5.4 $\mu$m in the fudosteine inhalation solution is not less than 50%.

Preferably, the percent mass of the particles with aerodynamic particle size of 1.4-5.4 $\mu$m in the fudosteine inhalation solution is not less than 53.2%, such as 54.9%, 55.1%, 63.5% and 67.9%.

Preferably, the percent mass of the particles with aerodynamic particle size of 2.1-5.4 $\mu$m in the fudosteine inhalation solution is not less than 41.5%, such as 44.6%, 46.7%, 49.4% and 50.3%.

Preferably, the inhalation solution has following droplet particle sizes during nebulization inhalation: D90 particle size of 7.0-9.5 $\mu$m, D50 particle size of 2.8-4.4 $\mu$m, and D10 particle size of 0.5-1.0 $\mu$m.

Preferably, the inhalation solution has droplet particle sizes during nebulization inhalation: D90 particle size of 7.13-9.42 $\mu$m, D50 particle size of 2.81-4.38 $\mu$m, and D10 particle size of 0.54-0.95 $\mu$m.

Preferably, the concentration of the active ingredient in the composition is 20-140 mg/ml.

Preferably, the concentration of the active ingredient in the composition is 80 mg/mL.

Preferably, the pH-adjusting agent is selected from the group consisting of hydrochloric acid, sulfuric acid, tartaric acid, lactic acid, citric acid, glacial acetic acid, malic acid, sodium citrate, sodium hydroxide, and a combination thereof.

Preferably, the driving gas flow rate of the nebulization inhalation composition during use is 4-10 L/min.

More preferably, the driving gas flow rate is 4-8 L/min.

An assembly of the fudosteine nebulised inhalation solution composition is provided according to another object of the present disclosure,

Wherein, the assembly comprises the aforementioned fudosteine nebulised inhalation solution composition and a nebulizing device for use in conjunction with the composition.

Wherein, the nebulizing device comprises a nebulizer and a nebulizing cup;

Preferably, the nebulizer has a compressor pressure parameter of 1.1-1.6 bar and a spraying rate of 0.4-0.6 ml/min, and the average particle diameter of the particles of the 0.9 wt% standard sodium chloride solution excited by the nebulizing cup is 2.5-3.5 $\mu$m.

Further preferably, the nebulizer has a compressor pressure parameter of 1.2-1.6 bar and a spraying rate of 0.5-0.6 ml/min.

Use of the fudosteine nebulised inhalation solution composition or the assembly comprising the fudosteine nebulised inhalation solution composition in the manufacture of a medicament product for expectorant treatment of respiratory diseases is provided according to another object of the present disclosure, wherein

(1) adding and dissolving fudosteine or a pharmaceutically acceptable salt thereof as an active ingredient into a solvent;

(2) adding a pH-adjusting agent into the solution of step (1) to adjust the pH of the solution to 3.7-3.9 to obtain an inhalation solution composition;

(3) filtering, filling and sealing the inhalation solution composition; and

(4) nebulizing the composition using a nebulizing device;

Preferably, the nebulizing device comprises an nebulizer and an nebulizing cup;

Preferably, the nebulizer has a compressor pressure parameter of 1.1-1.6 bar and a spraying rate of 0.4-0.6 ml/min, and the average particle diameter of the particles of the 0.9 wt% standard sodium chloride solution excited by the nebulizing cup is 2.5-3.5 $\mu$m.

Preferably, the administration site of the medicament product includes one or more of nose, throat, trachea, esophagus and main bronchus; and more preferably, the administration site of the medicament products is trachea and main bronchus.

Preferably, the fudosteine medicament product is used for expectorant treatment of one or more of bronchial asthma, chronic asthmatic bronchitis, bronchiectasis, pulmonary tuberculosis, pneumoconiosis, chronic obstructive emphysema, atypical mycobacteriosis, pneumonia and diffuse bronchitis.

Pharmacokinetic tests showed that the peak time of fudosteine in plasma and various tissues (lung, trachea, main bronchus, throat, tongue, nose, spleen and brain) after nebulization administration is shorter than that after oral administration, and fudosteine is more quickly and widely distributed in various tissues in vivo faster than that of oral administration. The peak concentration of nebulization administration in trachea, main bronchus, esophagus, throat, nose and lung is significantly higher than that of oral administration, especially in trachea and main bronchus; and the peak concentration in plasma, liver, heart, spleen and kidney is lower than that of oral administration. Therefore, compared with oral administration of fudosteine tablets, the fudosteine nebulization administration has a higher peak concentration in respiratory tissues and organs and a lower peak concentration in metabolic and excretory organs. The half-life of the fudosteine nebulised inhalation solution in plasma, lung, main bronchus, liver and brain is shorter than that of oral fudosteine tablets, and the half-life in esophagus is longer than that of oral fudosteine tablets. There is no obvious accumulation in plasma and tissue after administration, and the concentration of fudosteine in tissue and plasma is lower than the minimum quantitative limit 24 hours after administration, and the elimination rate is faster.

In the present disclosure, the dosage of the fudosteine nebulised inhalation solution composition is 2-5 mL. The results of animal experiments showed that the minimum effective dose of that fudosteine nebulised inhalation solution composition is obviously smaller than that of commercial fudosteine oral solution.

The present disclosure has the beneficial effects of:

(1) According to the characteristics of fudosteine in treating tracheal and main bronchial diseases, the present disclosure designed a fudosteine nebulised inhalation solution composition and a compression nebulizing device (nebulizer and nebulizing cup) for use in conjunction with the fudosteine nebulised inhalation solution composition, at the same time, limits and adjustments were made to the pressure parameters of the nebulizer and the output parameters of the nebulizing cup, and corresponding controls were made to the droplet particle size and particle size parameters (D10, D50 and D90) of the fudosteine nebulised inhalation solution composition, so as to reduce the residue of drug solution, improve the drug delivery rate and total delivery amount, and improve the drug deposition rate of fudosteine nebulization particles in trachea and main bronchus at the same time, that is, fudosteine nebulization particles can directly act on trachea and main bronchus, thus achieving the most ideal expectorant treatment effect.

(2) The fudosteine nebulised inhalation solution composition disclosed according to the present disclosure has special physical properties, and compared with similar fudosteine nebulization inhalation preparations, it overcomes the defects of wide droplet distribution, long treatment time and poor compliance of similar nebulization inhalation products.

(3) Through reasonable ingredient combination, the drug prescription of the present disclosure does not contain stabilizers such as EDTA, thus avoiding the risk of adverse reactions such as cough and asthma induced by medication.

(4) Compared with similar commercially available oral preparations of fudosteine, the fudosteine nebulised inhalation solution composition disclosed according to the present disclosure has the advantages of small effective dose, fast acting, smaller total drug dose when taken, and better medication safety.

## BRIEF DESCRIPTION OF DRAWINGS

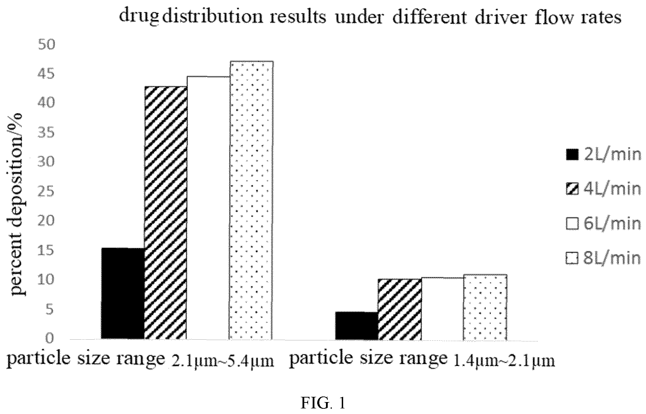

FIG. 1 shows the results of drug distribution under different driver flow rates.

## DETAILED DESCRIPTION

In order to further illustrate the purpose, technical solutions and beneficial effects of the present disclosure, the fudosteine nebulised inhalation solution composition, assembly and use thereof provided according to the present disclosure will be described in detail below in combination with specific embodiments. The described embodiments and examples are only exemplary and do not represent all technical solutions of the application. Based on the examples of the present disclosure, other embodiments obtained by those skilled in the art without any creative work also fall into the protection scope of the present disclosure.

## I. Interpretation of terms

The specific abbreviations used in the present disclosure are as follows: "FPD" is the abbreviation of fine particle dose; "MMAD" is the abbreviation of mass median aerodynamic diameter; GSD is the abbreviation of geometric standard deviation; and NGI is the abbreviation of Next Generation Impactor.

The term "particle size" may refer to a measurements made on a single particle or particle distribution. These parameters can be measured by various techniques, including dynamic light scattering, static light scattering, laser diffraction, deposition, time of flight, or other methods known to those skilled in the art. The particle size distribution can also be quantified by the size corresponding to a certain percentage distribution (Dx), where a certain percentage (X) of the population is smaller than the defined size. For example, a distribution with a D90 value of 500 nm means that 90% of the distribution (based on volume) has a size less than 500 nm.

## II. Experimental conditions

In the following examples, unless otherwise specified, the experimental operating conditions are as follows:

## Chromatographic conditions

Chromatographic column: C18, $4.6 \times 150$ mm, 5 $\mu$m;

Mobile phase: 5 mmol/L phosphoric acid solution of sodium heptane sulfonate $(1 \rightarrow 1000)$- methanol (90:10)

Detection wavelength: 210 nm; column temperature: 50 °C; injection volume: 10 $\mu$l;

Flow rate: 1.0 ml/min;

Solvent: water

## Preparation of standard curve

Preparation of reference stock solution: 100 mg of fudosteine reference was taken, weighed accurately, and placed in a 100 mL volumetric flask, dissolved and diluted to scale with mobile phase, and shaken evenly as the stock solution.

Preparation of standard curve: an appropriate amount of stock solution was accurately pipetted and quantitatively diluted with water step by step to prepare standard curve solutions with concentrations of about 16 μg/ml, 40 μg/ml, 80 μg/ml, 96 μg/ml and 160 μg/ml.

10 μL of each series of solutions was accurately measured, injected into the liquid chromatographer respectively, and linear regression with concentration as abscissa and peak area as ordinate was performed according to the least square method to obtain the linear regression equation: y = ax+b.

## Method for dose determination of fine particles

Method basis: the aerodynamic characteristics determination method of fine particles of inhalation solution composition (China Pharmacopoeia, 2020 edition, Part IV general rules 0951) and NGI(ApparatusE) collected by EP.

The specific operation is as follows:

(1) NGI was placed in a constant temperature box (or refrigerator) at 5 (±3) °C for at least 90 min; and the compressed nebulizing inhaler was preheated.

(2) First, NGI was assembled and connected with the leak detector, the pressure of the leak detector was set to 4 KPa, and the leak rate test was started. If the pressure was stable within 60 seconds, it means that the device is well sealed and can be performed for test.

(3) The nebulizer, NGI, high-capacity vacuum pump and critical flow controller was connected in turn from left to right; then the air flow meter was connected to detect the constant flow rate, the high-capacity vacuum pump and critical flow control was turned on and the knob adjusted to stabilize the flow rate.

(4) The critical flow control time parameter was set to 305 seconds.

(5) 2.5 mL of sample was taken, added into the nebulizing cup, and the compressed nebulizing inhaler and the "RUN" key of critical flow control was turned on at the same time. When the test reaches 300 seconds, the compression nebulizing inhaler was turn off, and the high-capacity vacuum pump and critical flow control was turned off after 5 seconds.

(6) the nebulizing cup, artificial larynx+adapter and stage 1-7 collecting trays (including filter tray) were cleaned and transferred respectively after the testing process was completely finished:

20 ml of water was accurately measured, added into the collection trays of Stage1-Stage6 respectively, mixed evenly, and then 1 ml was accurately measured and placed into a 10 ml volumetric flask, water was added to quantitatively to dilute to the scale, and shaken evenly to serve as the test solution of Stage1-Stage6; 50 ml of water was accurately measured, added into the Stage7 collection tray, and mixed evenly as the test solution of Stage7. MOC layer filter paper was taken, placed in a beaker, 50 ml of water was accurately measured and placed in the beaker. The filter paper was squeezed with a glass rod and stirred evenly, filtered, and the filtrate was taken as the MOC test solution. The mouth and throat device was taken, with one end sealed. 50 ml of water was measured accurately, placed in the mouth and throat, and shaken evenly as the mouth and throat test solution. The nebulizing cup was cleaned with proper amount of water, transferred to a 100 ml volumetric flask, water was added to dilute to the scale, shaken evenly, then was accurately transferred to a 1 ml to 10 ml volumetric flask. Water was added to dilute to the scale, and shaken evenly as the residual solution of the cup. 10 μL of each sample solution to be tested was accurately measured, injected into the liquid chromatographer respectively, and the chromatogram was recorded. The peak area A of each sample to be measured was substituted into the linear equation y=ax+b, and the concentration (c) of each stage was worked out and multiplied by the dilution multiple (f) of the sample solution, and the amount (m) of fudosteine in each stage was calculated.

Calculation formula: the mass of fudosteine: $m = \dfrac{(A-b)}{a} \times f = cf$

Where: m refers to the mass of fudosteine in the sample to be tested, mg;

A refers to the peak area of fudosteine in the sample to be tested;

c refers to the concentration of fudosteine in the sample to be tested;

f refers to the dilution multiple of the sample to be tested;

The measurement results of samples at all stages of particle size distribution were imported into CITDAs software one by one, and the corresponding results of each index were obtained.

**Method for determination of the delivery rate and the total delivery**

According to the "Determination of Delivery Rate and Total Delivery Amount" (China Pharmacopoeia, 2020 Edition, Part IV General Rules 0111) under the item of inhalation liquid preparations, the parameters of the respiratory simulator were set according to the respiratory characteristics.

For the test solution, the parameters of the respiratory simulator were set according to the adult respiratory characteristics. 2.5 mL of the sample was accurately measured, placed in an nebulizing cup, and the respiratory simulator was connected with filter paper, nebulizer and nebulizing cup device. At the same time, the nebulizer and respiratory simulator were turned on, nebulized for 1 min, the nebulizer was stopped, filter paper ① was collected, and placed in a 250 mL beaker. New filter paper was replaced, nebulized for another 10 min, filter paper ② was collected, and placed in a second 250 mL beaker. 40 ml of water was accurately measured, put in the first beaker, the filter paper was fully soaked and kept below the liquid level. After ultrasonic for 10 min, the filter paper was squeezed using a glass rod and stirred evenly, and then filtered; 2 ml of the continuous filtrate was accurately measured, placed in a 10 ml volumetric flask, diluted to scale with water, shaken evenly, filtered, and the continuous filtrate was take. 50 ml of water was accurately measured, placed in the first beaker, the filter paper was fully soaked and kept below the liquid level, after ultrasonic for 10 min. The filter paper was squeezed using a glass rod and stirred evenly, and then filtered. 1 ml of the continuous filtrate was accurately measured, placed in a 10 ml volumetric flask, diluted to scale with water, shaken evenly, filtered and the continuous filtrate was taken.

Determination method: each standard curve solution, filter paper ① test solution and filter paper ② test solution were accurately measured, injected into the liquid chromatographer respectively, and the chromatogram was recorded. The amount of fudosteine collected by each filter paper was calculated by peak area according to the standard curve method. The ratio of the amount of fudosteine collected by the first filter paper to the nebulization time is the delivery rate, and the total amount of fudosteine collected by each filter paper is the total delivery amount.

Unless otherwise specified, the formula of the fudosteine nebulised inhalation solution composition used in the examples is selected from the following formulas 1-3;

Formula 1: fudosteine (80 mg/mL), hydrochloric acid (appropriate amount), sodium hydroxide (appropriate amount if necessary) and water for injection. Hydrochloric acid and sodium hydroxide were added to adjust the pH value.

Formula 2: fudosteine (80 mg/mL), tartaric acid (appropriate amount), sodium hydroxide (appropriate amount if necessary) and water for injection. Tartaric acid and sodium hydroxide were added to adjust the pH value.

Formula 3: fudosteine (80 mg/mL), EDTA (0.5 mg/mL), hydrochloric acid (appropriate amount), sodium hydroxide (appropriate amount if necessary) and water for injection. Hydrochloric acid and sodium hydroxide were added to adjust the pH value.

Unless otherwise specified, the formula of the fudosteine nebulised inhalation solution composition was selected as formula 1;

Unless otherwise specified, the fudosteine nebulised inhalation solution composition was nebulized under the nebulization parameters of: nebulizer model of PARI BOY; nebulizer pressure of 1.2 bar; nebulizing cup model of PARI LC SPRINT; spraying rate of 0.6 ml/min.

The fudosteine nebulised inhalation solution compositions of the Formula 1 and the Formula 2 comprises were prepared by steps of:

S1: 50%-80% water volume of water for injection was added into the solution preparation container, the temperature of water was controlled at 25±10 °C. Nitrogen was filled into the water for injection for protection until the solution preparation was finished. The positive pressure of nitrogen was kept in the solutions preparation tank, and subsequent steps were carried out after determining the residual oxygen < 2 mg/L;

S2, the solid medicament was slowly added according to the formula, and stirred until it was completely dissolved;

S3, a pH-adjusting agent was added to adjust the pH, and simultaneously the residual oxygen was measured to < 2 mg/L, then the next step was carried out;

S4, water for injection was added to the full amount, and stirred until the mixture was uniform;

S5, filtering was performed with a membrane, and the solution was filled into an ampoule bottle and nitrogen was filled.

The fudosteine nebulised inhalation solution composition of formula 3 was prepared by the same steps as in formulas 1 and 2, except that in formula 3, the step S5 was as follows: filtering was performed with a membrane, and the solution was filled into an ampoule bottle.

### III. verification results

**Application Example 1:**

According to formulas 1-3 and the preparation method of the fudosteine nebulised inhalation solution composition, the fudosteine nebulised inhalation solution composition was prepared, the pH was adjusted to 3.7, and its impurities and formula stability were determined. The results are shown in Tables 1-3.

Table-1 Stability of Formula 1

| Test items | | Day 0 | Long-term, 12 months | Long-term, 24 months |
|---|---|---|---|---|
| Character | | meet the requirements | meet the requirements | meet the requirements |
| Identification | | retention time is consistent | retention time is consistent | retention time is consistent |
| pH | | 3.7 | 3.8 | 3.7 |
| Clarity and color of solution | | clear and colorless | clear and colorless | clear and colorless |
| Related substances | L-cystine | not detected | 0.02% | not detected |
| | Other single impurity | 0.06% | 0.04% | 0.05% |
| | Total impurity | 0.13% | 0.22% | 0.18% |
| S-isomer | | 0.41% | 0.67% | 0.62% |
| Loading | | meet the requirements | meet the requirements | meet the requirements |
| Visible foreign matters | | meet the requirements | meet the requirements | meet the requirements |
| Insoluble microparticles | | meet the requirements | meet the requirements | meet the requirements |
| Delivery rate and total delivery amount | | delivery rate : 12.87mg/ml total delivery amount : 41.86mg | delivery rate : 11.93mg/ml total delivery amount : 41.40mg | delivery rate : 11.13 mg/ml total delivery amount : 45.24 mg |
| Aseptic | | meet the requirements | meet the requirements | meet the requirements |
| Content | | 99.7% | 100.6% | 101.9% |

Table-2 Stability of Formula 2

| Test items | | Day 0 | Long-term, 12 months | Long-term, 24 months |
|---|---|---|---|---|
| Character | | meet the requirements | meet the requirements | meet the requirements |
| Identification | | retention time is consistent | retention time is consistent | retention time is consistent |
| pH | | 3.7 | 3.7 | 3.7 |
| Clarity and color of solution | | clear and colorless | clear and colorless | clear and colorless |
| Related substances | L-cystine | not detected | 0.02% | 0.01% |
| | Other single impurity | 0.06% | 0.07% | 0.06% |
| | Total impurity | 0.13% | 0.24% | 0.21% |
| S-isomer | | 0.51% | 0.70% | 0.75% |

(continued)

| Test items | Day 0 | Long-term, 12 months | Long-term, 24 months |
|---|---|---|---|
| Loading | meet the requirements | meet the requirements | meet the requirements |
| Visible foreign matters | meet the requirements | meet the requirements | meet the requirements |
| Insoluble microparticles | meet the requirements | meet the requirements | meet the requirements |
| Delivery rate and total delivery amount | delivery rate : 11.92mg/ml total delivery amount : 41.36mg | delivery rate : 11.28mg/ml total delivery amount : 42.35mg | delivery rate : 11.23 mg/ml total delivery amount : 43.14 mg |
| Aseptic | meet the requirements | meet the requirements | meet the requirements |
| Content | 100.2% | 100.8% | 101.5% |

Table-3 Stability of Formula 3

| Test items | | Day 0 | Long-term, 12 months | Long-term, 24 months |
|---|---|---|---|---|
| Character | | meet the requirements | meet the requirements | meet the requirements |
| Identification | | retention time is consistent | retention time is consistent | retention time is consistent |
| pH | | 3.7 | 3.7 | 3.7 |
| Clarity and color of solution | | clear and colorless | clear and colorless | clear and colorless |
| Related substances | L-cystine | not detected | 0.01% | 0.01% |
| | Other single impurity | 0.04% | 0.06% | 0.04% |
| | Total impurity | 0.15% | 0.20% | 0.22% |
| S-isomer | | 0.44% | 0.66% | 0.69% |
| Loading | | meet the requirements | meet the requirements | meet the requirements |
| Visible foreign matters | | meet the requirements | meet the requirements | meet the requirements |
| Insoluble microparticles | | meet the requirements | meet the requirements | meet the requirements |
| Delivery rate and total delivery amount | | delivery rate : 12.07mg/ml total delivery amount : 42.34mg | delivery rate : 12.21mg/ml total delivery amount : 42.83mg | delivery rate : 11.96 mg/ml total delivery amount : 46.21 mg |
| Aseptic | | meet the requirements | meet the requirements | meet the requirements |
| Content | | 99.9% | 101.1% | 100.5% |

The results of formula preparations showed that by adjusting the preparation process, the product could be protected from oxidation by adopting measures such as nitrogen filling in the solution preparation process to exclude oxygen, nitrogen filling in the filling process and nitrogen filling in the packaging bag, which was basically consistent with the stability of the formula with EDTA. It showed that the fudosteine nebulised inhalation solution composition produced by the preparation process according to the present disclosure had good stability.

**Application Example 2:**

The experimental conditions of different driving gas flow rates were selected to test their influence on the nebulization effect when applied to the fudosteine inhalation solution composition. Remarkably, the inventors found through a large number of parallel experiments that when the pH of the fudosteine inhalation solution composition was in the range of 2.5-5.0, there was no significant difference in the nebulization effect at the same driving flow rate. In order to further explain the present disclosure, the specific pH of the fudosteine inhalation solution composition in this Application Example was selected as 4.0. The drug distribution was shown in FIG. 1, and the test results are shown in Table 4:

Table 4 Effect of different driving gas flow rates on drug nebulization results by spray nebulization

| | Serial number 1 | Serial number 2 | Serial number 3 | Serial number 4 |
|---|---|---|---|---|
| Driving gas flow rate | 2L/min | 4L/min | 6L/min | 8L/min |
| Delivery rate (mg/min) | 3.42 | 12.26 | 13.11 | 13.62 |
| Total delivery amount (mg) | 13.57 | 45.91 | 48.54 | 51.29 |
| Percent deposition (%) (particle size range1.4$\mu$m-5.4$\mu$m) | 20.3 | 53.2 | 55.3 | 58.4 |
| MMAD ($\mu$m) | 9.286 | 4.852 | 4.263 | 3.951 |
| Percent deposition (%) (particle size range2.1$\mu$m-5.4$\mu$m) | 15.5 | 42.9 | 44.6 | 47.2 |
| Percent deposition (%) (particle size range1.4$\mu$m-2.1$\mu$m) | 4.8 | 10.3 | 10.7 | 11.2 |

The above results showed that the delivery rate, total delivery amount and percent deposition (particle size range 1.4 $\mu$m-5.4 $\mu$m) increased with the increase of driving gas flow rate, while the median particle size MMAD decreased continuously. At 2 L/min, the delivery rate, total delivery and percent aerosol deposition were all too small, and the percent deposition of particles (particle size range 2.1 $\mu$m-5.4 $\mu$m) which tend to be deposited in the main bronchus and trachea and particles (particle size range 1.4 $\mu$m-2.1 $\mu$m) which tend to be deposited in the lung were obviously lower than 4-8 L/min. Clinical trials showed that particles of 5-10$\mu$m were generally deposited in the upper respiratory tract, and particles of 1-5 $\mu$m were deposited in the lower respiratory tract. Therefore, because the drugs inhaled by nebulization need to be deposited in the lower respiratory tract, the median particle size MMAD of more than 5 $\mu$m could not meet the general requirements of nebulization inhalation. When the driving gas flow rate was greater than 4 L/min, the delivery rate, total delivery, percent aerosol deposition (particle size range of 1.4 $\mu$m-5.4 $\mu$m), the percent deposition of particles with a size range of 2.1 $\mu$m-5.4 $\mu$m and the percent deposition of particles with a size range of 1.4 $\mu$m-2.1 $\mu$m increased continuously. Although serial number 3 and serial number 4 increased in the range of 6-8 L/min, the increase was not significant, which could meet the general requirements of drug delivery for nebulization inhalation.

**Application Example 3:**

Different brands of nebulizers and nebulizing cups were selected to investigate the effective deposition dose of fudosteine nebulised inhalation solution composition. Remarkably, the inventors found through a large number of parallel experiments that when the pH of the fudosteine inhalation solution composition was in the range of 2.5-5.0, there was no significant difference in the effective deposition dose when using the same nebulizer and nebulizing cup. In order to further explain the present disclosure, the specific pH of the fudosteine inhalation solution composition in this Application Example was selected as 5.0, and the sample prescriptions used were formula 1 and formula 2.

The nebulization tests were carried out on the samples, and the nebulizer and nebulizing cup used in the experiment can both be purchased in the market. The purchased nebulizer models include PARI BOY SX, Yuyue 403E, Yuyue 403C, Yuyue 403M, Yinghua Rongtai FA and Yinghua Rongtai HA03; Nebulizing cup models include PARI LC SPRINT series blue core, PARI LC PLUS and Yinghua Rongtai hand-held blue respiratory valve.

In Examples 1-5 and Comparative Examples 1-5 of the present application, the formulas of the inhalable solution composition of the present disclosure were selected to investigate the influence of the parameters of different nebulizers and nebulizing cup assemblys on the nebulization properties. The parameter conditions of Examples 1-5 are shown in Table 5, and the parameter conditions of Comparative Examples 1-5 are shown in Table 6:

Table 5 Formulas and nebulizing Device Assemblys of Examples 1-5 of the present disclosure

| | | Example 1 | Example 2 | Example 3 | Example 4 | Example 5 |
|---|---|---|---|---|---|---|
| Prescriptions of inhalable solution composition | | Formula 1 | Formula 2 | Formula 1 | Formula 2 | Formula 2 |
| Nebulizers | Manufacturer and model | PARI BOY | | Yinghua Rongtai HA03 | Yuyue 403C | PARI BOY |
| | Pressure (bar) | 1.1 | 1.2 | 1.4 | 1.4 | 1.6 |
| Nebulizing cups | Manufacturer and model | PARI LC SPRINT | PARI LC PLUS | Yinghua Rongtai B06 Narrow bite type | Yuyue 403 | PARI SPRINT |
| | Spraying rate (ml/min) | 0.4 | 0.4 | 0.5 | 0.6 | 0.6 |
| | Average particle diameter (MMAD)/$\mu$m | 3.2 | 3.4 | 2.5 | 3.0 | 3.5 |
| | Percentage of droplets with a particle diameter less than 5 microns(0.9 wt% sodium chloride standard solution) | 71% | 64% | 68% | 65% | 63% |
| Note: Average particle diameter (MMAD) refers to the average particle diameter of particles excited in the 0.9 wt% standard sodium chloride solution. | | | | | | |

Table 6 Formulas and nebulization parameters of Comparative Examples 1-5

| | | Comparative example 1 | Comparative example 2 | Comparative example 3 | Comparative example 4 | Comparative example 5 |
|---|---|---|---|---|---|---|
| Formulas of inhalable solution composition | | **Formula 1** | **Formula 2** | **Formula 1** | **Formula 2** | **Formula 2** |
| Nebulizers | Pressure(bar) | 0.8 | 1.1 | 1.2 | 2.0 | 1.6 |
| Nebulizin g cups | Spraying rate (ml/min) | 0.4 | 0.35 | 0.5 | 0.6 | 0.6 |
| | Average particle diameter ($\mu$m) | 3.2 | 3.2 | 1.5 | 3.5 | 5.0 |
| | Percentag e of droplets with a particle diameter less than 5 microns | 62% | 62% | 82% | 65% | 50% |

The nebulization performance of the above examples and comparative examples was measured, and the results are shown in Table 7:

Table 7 Aerodynamic Particle Size and Distribution under Different Nebulizers

| No.<br>Parameters and results | | Example 1 | Example 2 | Example 3 | Example 4 | Example 5 | Comparative example 1 | Comparative example 2 | Comparative example 3 | Comparative example 4 | Comparative example 5 |
|---|---|---|---|---|---|---|---|---|---|---|---|
| Droplet particle size | $D_{10}$ (μm) | 0.95 | 0.84 | 0.78 | 0.64 | 0.54 | 1.45 | 1.66 | 0.47 | 0.44 | 1.35 |
| | $D_{50}$ (μm) | 4.38 | 3.85 | 3.51 | 3.29 | 2.81 | 4.82 | 4.87 | 2.82 | 2.73 | 4.72 |
| | $D_{90}$ (μm) | 9.42 | 9.24 | 8.67 | 8.22 | 7.13 | 10.87 | 10.45 | 6.88 | 6.91 | 11.07 |
| Nebulization characteristic result | Percent deposition (%) (particle size range 1.4-5.4 μm) | 63.5 | 53.2 | 54.9 | 55.1 | 67.9 | 37.7 | 39.2 | 29.1 | 32.8 | 38.0 |
| | Percent deposition (%) (particle size range 2.1-5.4 μm) | 50.3 | 44.6 | 41.5 | 46.7 | 49.4 | 32.6 | 33.4 | 8.9 | 6.2 | 30.8 |
| | Percent deposition (%) (particle size range 1.4 -2.1 μm) | 13.2 | 8.6 | 13.4 | 8.4 | 18.5 | 5.1 | 5.8 | 20.2 | 26.6 | 7.2 |
| | MMAD (μm) | 4.222 | 3.483 | 3.459 | 3.538 | 2.305 | 5.219 | 5.524 | 1.835 | 1.860 | 5.231 |
| | GSD | 2.21 | 2.26 | 2.03 | 2.10 | 2.17 | 2.151 | 2.001 | 2.216 | 2.265 | 2.014 |
| | Delivery rate (mg/min) | 9.55 | 9.80 | 10.41 | 11.24 | 11.97 | 9.21 | 7.32 | 12.08 | 12.80 | 12.51 |
| | Total delivery amount (mg) | 52.23 | 54.90 | 56.63 | 59.61 | 60.74 | 49.11 | 42.06 | 61.02 | 62.02 | 61.32 |

It can be seen from Table 7 that there was a certain linear relationship between $D_{50}$ and MMAD, and MMAD also increased with the increase of $D_{50}$. Examples 1-5 had higher percent deposition (particle size range of 1.4 μm-5.4 μm) and delivery rate. Particles which tend to be deposited in trachea and main bronchus (particle size range of 2.1 μm-5.4 μm) and particles which tend to be deposited in lung (particle size range of 1.4 μm-2.1 μm) had higher percent of deposition, and their MMAD values were less than 5 μm and relatively lowest. However, the MMAD values of Comparative Example 1, Comparative Example 2 and Comparative Example 5 were more than 5 μm, and it was difficult to form effective deposition in trachea and lung. In Comparative Example 3 and Comparative Example 4, the MMAD was smaller, and the particles were mainly deposited in alveoli, so it was difficult to exert the pharmacological effect on trachea.

Based on the above experimental results, for the fudosteine nebulised inhalation solution composition of the present disclosure combining the compressor pressure of the nebulizer of the compression nebulizing device for use in conjunction with it, the spraying rate and the average particle diameter of the particles excited by the 0.9 wt% standard sodium chloride solution in the nebulizing cup were controlled, so that the droplet particle size parameters of the fudosteine nebulised inhalation solution composition were controlled within a certain range, that is, the droplet particle size was as follows: the D90 particle size was 7.0-10.0 μm, and the D50 particle size was 2.8-4.5 μm, and the D10 particle size was 0.5-1.5 μm, and the percent deposition of the particles with the aerodynamic particle size of 2.1-5.4 μm in the fudosteine inhalation solution was not less than 41.5%, which improved the drug delivery rate and total delivery amount, and enabled nebulization particles to directly act on trachea and main bronchus, thus achieving the most ideal expectorant treatment effect.

**Application Example 4:**

The pharmacokinetic characteristics and distribution of the fudosteine inhalation solution (Formula 1) in plasma and tissues were investigated by single and continuous nebulization administration of fudosteine inhalation solution in the rats. By comparing with the distribution of fudosteine tablets (original dosage form) in the tissue by single and continuous oral administration, the tissue distribution characteristics of fudosteine inhalation solutionare were illustrated. The results are shown in Table 8:

Table 8 Comparison of exposure of fudosteine in plasma and tissues after single or continuous administration of low-dose fudosteine inhalation solution and fudosteine tablets (original dosage form) to SD rats.

| Tissues | Parameters | Single fudosteine inhalation solution | Multiple fudosteine inhalation solution | Ratio (Multiple /single) | Single original dosage form | Multiple (original dosage form | Ratio (Multiple /single) | Ratio of single (Fudosteine inhalation solution / original dosage form) | Ratio of multiple (Fudosteine inhalation solution / original dosage form) |
|---|---|---|---|---|---|---|---|---|---|
| plasma | Tmax (hr) | 0.083 | 0.083 | - | 0.500 | 0.500 | - | - | - |
| | Cmax (ng/mL) | 6730 | 8130 | 1.21 | 27250 | 12388 | 0.455 | 0.247 | 0.656 |
| | AUC0-t (hr*ng/mL) | 12621 | 10023 | 0.794 | 56744 | 18367 | 0.324 | 0.222 | 0.546 |
| | T1/2 (hr) | 1.10 | 0.748 | - | 1.07 | 1.66 | - | - | - |
| Lung | Tmax (hr) | 0.083 | 0.083 | - | 0.500 | 0.500 | - | - | - |
| | Cmax (ng/mL) | 16588 | 19182 | 1.16 | 13694 | 9844 | 0.719 | 1.21 | 1.95 |
| | AUC0-t (hr*ng/g) | 16860 | 10113 | 0.600 | 25466 | 17467 | 0.686 | 0.662 | 0.579 |
| | T1/2 (hr) | 1.15 | 0.370 | - | 1.24 | 1.09 | - | - | - |
| Trachea | Tmax (hr) | 0.083 | 0.083 | - | 0.500 | 0.500 | - | - | - |
| | Cmax (ng/mL) | 148700 | 341858 | 2.30 | 33325 | 24775 | 0.743 | 4.46 | 13.8 |
| | AUC0-t (hr*ng/g) | 100654 | 143946 | 1.43 | 59462 | 41939 | 0.705 | 1.69 | 3.43 |
| | T1/2 (hr) | 0.83 | 0.959 | - | 1.34 | 1.27 | - | - | - |
| Main bronchus | Tmax (hr) | 0.083 | 0.083 | - | 0.500 | 0.500 | - | - | - |
| | Cmax (ng/mL) | 56475 | 91008 | 1.61 | 12303 | 9088 | 0.739 | 4.59 | 10.0 |
| | AUC0-t (hr*ng/g) | 22777 | 28774 | 1.26 | 28486 | 5908 | 0.207 | 0.800 | 4.87 |
| | T1/2 (hr) | 0.25 | 0.204 | - | 1.79 | 0.79 | - | - | - |
| Esophagus | Tmax (hr) | 0.083 | 0.083 | - | 0.083 | 0.083 | - | - | - |
| | Cmax (ng/mL) | 312625 | 200319 | 0.641 | 51781 | 38613 | 0.746 | 6.04 | 5.19 |
| | AUC0-t (hr*ng/g) | 131164 | 113448 | 0.865 | 41110 | 32017 | 0.779 | 3.19 | 3.54 |
| | T1/2 (hr) | 1.58 | 2.73 | - | 1.28 | 1.04 | - | - | - |
| throat | Tmax (hr) | 0.083 | 0.083 | - | 0.500 | 0.500 | - | - | - |
| | Cmax (ng/mL) | 59400 | 40438 | 0.681 | 15058 | 16950 | 1.13 | 3.94 | 2.39 |
| | AUC0-t (hr*ng/g) | 39145 | 35896 | 0.917 | 29287 | 32822 | 1.12 | 1.34 | 1.09 |
| | T1/2 (hr) | 1.11 | 1.01 | - | 1.47 | 1.23 | - | - | - |

The data in Table 8 showed that, for oral administration, the exposure of fudosteine in plasma was higher; for nebulization administration, the exposures of fudosteine in various tissues were all higher than that in plasma, and the exposure of nebulization administration in respiratory systems such as throat and trachea was higher than that of oral administration. The exposure of single nebulization administration in throat, trachea and esophagus was 1.34-3.19 times that of oral administration, and that in other tissues was lower than that of oral administration. The exposure (AUC0-t (hr*ng/g)) of multiple nebulization administration in the nose, throat, trachea, esophagus and main bronchus was 1.00-4.87 times that of oral administration, especially the exposure in trachea and main bronchus was 3.43 times and 4.87 times that of oral administration, and that in other tissues was lower than that of oral administration, thus further explaining that the present disclosure can enable fudosteine to act better on trachea and main bronchus.

The peak time of fudosteine in plasma and various tissues (lung, trachea, main bronchus, throat, tongue, nose, spleen, brain) after nebulization administration was shorter than that after oral administration, and it was widely distributed in various tissues in vivo faster than that of fudosteine tablets after oral administration.

The peak concentration of nebulization administration in trachea, main bronchus, esophagus, throat, nose and lung was significantly higher than that of oral administration, and the peak concentration in plasma, liver, heart, spleen and kidney was lower than that of oral administration. Compared with the oral administration of fudosteine tablets, nebulization administration has a higher peak concentration in respiratory system tissues and organs and a lower peak concentration in metabolism and excretion organs.

The half-life of fudosteine nebulised inhalation solution in plasma, lung, main bronchus, liver and brain was shorter than that of oral fudosteine tablets, and the half-life in esophagus was longer than that of oral fudosteine tablets, and the half-life in other tissues (trachea, throat, tongue, nose, heart, spleen and kidney) was similar.

There was no obvious accumulation in plasma and tissues after continuous nebulization of fudosteine inhalation solution and continuous oral administration of fudosteine tablets, and the concentration of fudosteine in tissues and plasma 24 hours after administration was lower than the minimum quantitative limit, and the elimination rate was fast.

**Application Example 5: comparison of pharmacokinetic parameters between oral and inhalation**

The pharmacokinetic characteristics of the fudosteine inhalation solution (Formula 1) in rats were investigated by single nebulization administration of fudosteine inhalation solution in the SD rats.

The absolute bioavailability of the fudosteine inhalation solution (Formula 1) in rats was investigated by comparing with intravenous injection of the fudosteine inhalation solution in SD rats.

The relative bioavailability of the fudosteine inhalation solution (Formula 1) in rats was investigated by comparing with the same amount of fudosteine tablets given orally to SD rats.

The results are shown in Table 9:

Table 9 Summary of average pharmacokinetic parameters of fudosteine in different administration forms

| Parameters | Injection | Oral | Nebulised inhalation |
|---|---|---|---|
| $T_{1/2}$ (h) | 0.824 | 0.840 | 0.928 |
| $T_{max}$ (h) | 0.083 | 0.438 | 2.08 |
| $C_{max}$ (ng/mL) | 48475 | 59275 | 7616 |
| $AUC_{0-t}$ (h·ng/mL) | 102008 | 113191 | 18313 |
| $AUC_{0-\infty}$ (h·ng/mL) | 102484 | 113459 | 18593 |
| ReLative BA (%) | - | - | 16.2 |
| AbsoLute BA (%) | - | 111 | 18.0 |

Pharmacokinetic results compared single administration of the two dosage forms.

Systemic exposure: after nebulization administration of fudosteine inhalation solution and oral administration of the same dose of fudosteine tablets, the exposure of fudosteine inhalation solution in blood was less than that of fudosteine tablets.

Bioavailability: comparing nebulization administration of fudosteine inhalation solution with oral administration of the same dose of fudosteine tablets, the relative bioavailability of fudosteine inhalation solution in rats was 16.2%±6.43%, the absolute bioavailability of fudosteine inhalation solution in rats was 18.0%±7.13%, and the absolute bioavailability of fudosteine tablets in rats was 111%±22.6%. The bioavailability of fudosteine inhalation solution was lower than that of fudosteine tablets.

Peak time and peak concentration: the fudosteine inhalation solution was absorbed into blood quickly, achieving a faster peak time in the blood than fudosteine tablets, and the peak concentration was lower.

Elimination half-life: there was no significant difference in the elimination half-life between the single nebulization administration of the fudosteine inhalation solution and the single oral administration of mid-dose fudosteine tablets.

**Application Example 6 Efficacy Experiment**

1. Test sample, reference drug and reagents

1.1. The test sample

Fudosteine inhalation solution (Formula 1): 80 mg/ml, colorless and clear liquid, self-made.

1.2. Control drug

1.2.1. Fudosteine tablets: 0.2 g/tablet, white tablets, purchased from Jiangsu Zhengdafenghai Pharmaceutical Co., Ltd., with batch numbers of 2002151 and 2105141 respectively, and expiration dates of July 2021 and April 2023 respectively.

Preparation: a certain amount of fudosteine tablets was taken into a glass container and ground into fine powder, appropriate amount of purified water was added, ground while water was added, transferred to a graduated test tube, ultra-pure water was added, mixed evenly, the volume was fixed, and solutions with concentrations of 68 mg/mL, 34 mg/mL, 17 mg/mL, 8.5 mg/mL and 4.25 mg/mL for rats to test were prepared respectively. A solution with a concentration of 3.5 mg/mL for mice to test was prepared, immediately before it was used.

1.2.2. Ambroxol hydrochloride oral solution: 100mL: 0.6 g, colorless to yellowish clear liquid, purchased from China Resources Sanjiu (Nanchang) Pharmaceutical Co., Ltd., with the batch number of 2003004J and an expiration date of February 2022.

Preparation: an appropriate amount of ambroxol hydrochloride oral solution was measured, an appropriate amount of ultrapure water was added and mixed evenly to prepare a solution with a concentration of 4.5 mg/mL, immediately before it was used.

1.2.3. Prednisolone acetate tablets: 5mg/tablet, white tablets, purchased from Shanghai Shangyao Xinyi Pharmaceutical Co., Ltd., with a batch number of 017200501 and an expiration date of May 2023.

Preparation: a certain amount of prednisolone acetate tablets was taken into a glass container and ground into fine powder, appropriate amount of purified water was added, ground while water was added, transferred to a graduated test tube, ultra-pure water was added, mixed evenly, the volume was fixed, and a solution with a concentration of 1.25 mg/mL was prepared, immediately before it was used.

1.2.4. Acetylcysteine tablets: 0.2g/tablet, white tablets, purchased from ZAMBON S.p.A, with a batch number of 1002217 and an expiration date of February 2023.

Preparation: a certain amount of acetylcysteine tablets was taken into a glass container and ground into fine powder, appropriate amount of purified water was added, ground while water was added, transferred to a graduated test tube, ultra-pure water was added, mixed evenly, the volume was fixed, and a solution with a concentration of 43.2 mg/mL was prepared, immediately before it was used.

1.2.5. Acetylcysteine solution for inhalation: colorless and clear liquid, purchased from ZAMBON S.p.A, with a batch number of 28005283 and an expiration date of May 2025.

1.3. Moulding agent

Lipopolysaccharide (LPS): white crystal, 100 mg/bottle, purity ≥99.5%, produced by SIGMA Company, with a batch number of 028M4094V and an expiration date of December 2023.

Preparation: 100 mg of lipopolysaccharide was weighed, physiological saline was added, mixed evenly, the volume was fixed to 100 mL, and a lipopolysaccharide solution with a concentration of 1mg/mL was prepared, immediately before it was used.

1.4. Reagents

1.4.1. Physiological saline: colorless and transparent liquid, produced by Shijiazhuang No.4 Pharmaceutical Co., Ltd., with a batch number of 2101031602 and the expiration date of November 2023.

1.4.2. Sodium hydroxide: sodium hydroxide: analytically pure, with a content of ≥96%, 500g/ bottle, purchased from BeiJing chemical works, with a batch number of 20160912 and the expiration date of November, 2022.

Preparation: 20 g of sodium hydroxide was weighed, ultrapure water was added, mixed evenly, the volume to 500 mL was fixed, and a solution (1 mol/L) with a concentration of 0.04 g/mLwas prepared, immediately before it was used.

Preparation: 1.8 mL of sodium hydroxide solution was measured and physiological saline was added to a constant volume of 100 mL, and the pH value was measured to 11.9 after mixing evenly, thus the alkaline sodium chloride solution was obtained.

1.4.3. Phenol red: analytically pure, with a content of ≥99%, 25 g/bottle, produced by Shandong Xiya Chemical Co., Ltd., with a batch number of B4301 and an expiration date of April, 2022.

Preparation: 2.5 g of phenol red was weighed and alkaline sodium chloride solution was added to a constant volume of 100 mL, the pH value was adjusted to 11.9 with sodium hydroxide solution, which was equal to the alkaline sodium chloride solution, and filtered with a 0.22 $\mu$m membrane.

Preparation of phenol red standard curve solution: 50 mg of phenol red was accurately weighed, alkaline sodium chloride solution was added to constant volume to 50 mL, filtered with a 0.22 $\mu$m membrane, and the concentration was 1000 $\mu$g/mL, and diluted with alkaline sodium chloride solution to 7 concentration solutions of 10 $\mu$g/mL, 5 $\mu$g/mL, 2.5 $\mu$g/mL, 1.25 $\mu$g/mL, 0.63 $\mu$g/mL, 0.31 $\mu$ g and 0.16 $\mu$g/mL.

1.4.4. Sodium tetraborate (borax, $Na_2B_4O_7 \cdot H_2O$): colorless and hard crystalline powder, analytically pure, content ≥99.5%, purchased from Tianjin Kaitong Chemical Reagent Co., Ltd. and an expiration date of September 2024.

Preparation: a certain amount of $Na_2B_4O_7 \cdot H_2O$ was weighed and dissolved in concentrated sulfuric acid to prepare a 25 mmol/L sodium tetraborate concentrated sulfuric acid solution.

1.4.5. Carbazole (C12H9N): colorless monoclinic flaky crystal, analytically pure, and purchased from Shanghai Yi'en Chemical Technology Co., Ltd., with a batch number of RH297875 and an expiration date of September 2024.

Preparation: 125 mg C12H9N was weighed and dissolved in 100 mL absolute ethanol to prepare 7.5 mmol/L carbazole solution.

1.4.6. Sodium chloride (NaCl): white tetragonal crystal or crystalline powder, analytically pure, with a content of ≥99.5%, purchased from Tianjin Zhiyuan Chemical Reagent Co., Ltd. with an expiration date of April 2023.

Preparation: 35.10 mg NaCl was accurately weighed, dissolved in 200 mL ultra-pure water, and mixed evenly to prepare a sodium chloride solution with a concentration of 3 mol/L.

1.4.7. D-glucuronic acid lactone (C6H8O6): white crystal or crystalline powder, analytically pure, with a content of ≥99%, purchased from Shanghai Yi'en Chemical Technology Co., Ltd., with a batch number of RH297877 and an expiration date of September 2024.

Preparation: 50 mg of C6H8O6 was accurately weighed, dissolved in 50 mL of 3 mol/L sodium chloride, and a solution with a concentration of 1 mg/mL was prepared.

## 2. Instruments

Small animal mouth and nose exposure system: HRH-MNE3026 Type, produced by Beijing Huironghe Technology Co., Ltd.

Microplate reader: Synergy HTX Type, produced by American Boten Instrument Co., Ltd.

PH meter: PB-10 Type, manufactured by Sartorius Company.

## 3. Animals

Mice: ICR species SPF grade; Rats: SD species SPF grade.

ICR mice, half male and half female, weighing 18-20 g, were purchased from Beijing Weitong Lihua Experimental Animal Technology Co., Ltd. with the license number SYXK (Beijing) 2016-0006; SD rats, half male and half female, weighing 200-220 g, 220-240 g, were purchased from Beijing Weitong Lihua Experimental Animal Technology Co., Ltd. with the license number SCXK (Beijing) 2016-0011.

## 4. Test process

### 4.1. Data analysis method

Administration route: nebulization inhalation.

The data produced in this test were analyzed by using SPSS software, and $P < 0.05$ indicated that the difference between the groups was statistically significant. In addition, the ED50 value was used to compare the effectiveness of fudosteine tablets and fudosteine inhalation solution.

### 4.2 Investigation of nebulization conditions

The nebulizing device of small animal mouth and nose exposure system was used for administration. After the aerosol concentration of the test sample reached steady state, the particle size was monitored and analyzed. Two exposure ports were randomly selected, and each exposure port was measured twice, and the average value was taken. The mass median diameter (MMAD) and geometric standard deviation (GSD) values of aerosol particles were read by a particle size detector. The glass fiber filter membrane was placed in the randomly selected exposure port, and tests were performed under two nebulization conditions. Three samples were taken under each condition. After the filter membrane was collected, the aerosol concentration was detected by LC-MS/MS method.

The results are shown in Table 10.

Table 10 Investigation Results of Nebulization Conditions

|  | **Nebulization condition 1** | **Nebulization condition 2** |
|---|---|---|
| Aerosol concentration (mg/l) | 0.812 | 0.624 |
| Average particle size of aerosol particles ($\mu$m) | 1.68 | 3.86 |
| D90 ($\mu$m) | 8.76 | 9.82 |
| D50 ($\mu$m) | 3.49 | 5.31 |
| D10 ($\mu$m) | 0.68 | 1.15 |

The results showed that under the nebulization condition 1, the average aerosol concentration was 0.812 mg/L, the average particle size of the generated aerosol particles was 1.68 $\mu$m (equivalent to average particle size of about 4.0 $\mu$m when it was measured by a new generation impactor NGI for humans), and the D50 was 3.49 $\mu$m, which met the particle size requirements of nebulized drugs deposited in the airway and lungs and was beneficial to exert their efficacy. However, the average particle size under nebulization condition 2 was larger (equivalent to average particle size of about 7.0 $\mu$m when it was measured by a new generation impactor NGI for humans).

4.3. Influence on airway mucus secretion

According to the characteristics of phenol red partially excreted from the airway after intraperitoneal injection into mice, the excretion of phenol red in trachea was determined to investigate the influence of the test sample on airway secretion. The higher excretion amount in the airway, the stronger the expectorant effect of fudosteine test sample.

4.3.1 Phenol red excretion test in mice

ICR species mice were randomly divided into 8 groups according to their body weight, that is, model control group, positive ambroxol hydrochloride group, fudosteine tablet group (70.8 mg/kg), high dose of fudosteine inhalation solution group (14.6 mg/kg), medium dose of fudosteine inhalation solution group (7.3 mg/kg), low dose of fudosteine inhalation solution group (3.65 mg/kg), and control group - high dose of fudosteine inhalation solution, with 14 animals in each group, half male and half female. Animals in each group were administered according to the dosage, nebulizer and route in Table 11, once a day for 3 consecutive days. Animals in each group were fasted for 16 h before the last administration. 30 min after the last administration, the animals in each group were intraperitoneally injected with 2.5% phenol red solution, and 30 min later the mice were killed by cervical dislocation. The trachea was cut open and put into 1.0 mL alkaline sodium chloride solution, and the ultrasonic vibration was carried out for 5 min. The excretion amount of phenol red in the airway was measured by microplate reader at the wavelength of 546 nm.

Table 11 Influence on Phenol Red Excretion in Mice ($\bar{x}$, n=14)

| Groups | Nebulization condition | Dosage (mg/kg) | Phenol red airway excretion amount (μg/mL) |
|---|---|---|---|
| Model control group | Nebulization condition 1 | / | 2.71 |
| Positive ambroxol hydrochloride group | | / | 4.35** |
| Fudosteine tablet group | | 70.8 | 2.65 |
| High dose of fudosteine inhalation solution group | | 14.6 | 3.72* |
| Medium dose of fudosteine inhalation solution group | | 7.3 | 3.25 |
| Low dose of fudosteine inhalation solution group | | 3.65 | 3.20 |
| Control group-high dose of fudosteine inhalation solution | Nebulization condition 2 | 15.3 | 3.09 |
| Control group-medium dose of fudosteine inhalation solution | | 7.65 | 2.65 |
| Control group-low dose of fudosteine inhalation solution | | 3.83 | 2.53 |

The results show that, compared with the model control group, the excretion amount of phenol red in trachea of mice in positive ambroxol hydrochloride group and high dose of fudosteine inhalation solution group increased significantly ($P < 0.01$; $P<0.05$). Although the excretion amount of phenol red in trachea of mice in fudosteine tablet group, medium dose of fudosteine inhalation solution group and low dose of fudosteine inhalation solution group showed an increasing trend, there was no statistical difference. Although the excretion amount of phenol red in trachea of mice in the control group - high dose of fudosteine inhalation solution producing larger nebulization particle size showed an increasing trend, there was no statistical difference.

At the same time, the nebulization conditions also affect the efficacy results. Although the efficacy of the administered dose of 15.3 mg/kg was achieved under nebulization condition 2, it was significantly inferior to that of 14.6 mg/kg under nebulization condition 1. There was no significant difference in efficacy between the middle and low dosage groups and the model control group. It showed that the nebulization conditions should be strictly met in accordance with the requirements when the animals were administered by nebulization.

4.3.2. Detection test of airway secretion and mucopolysaccharide content in rats with mucus hypersecretion induced by LPS.

SD species rats were randomly divided into 11 groups according to their body weight, that is, - nebulization blank control group for normal animal, - positive acetylcysteine solution for inhalation group for normal animal, - high dose of fudosteine inhalation solution group (8.75 mg/kg) for normal animal, - medium dose of fudosteine inhalation solution group (4.5 mg/kg) for normal animal, - low dose of fudosteine inhalation solution group (2.25 mg/kg) for normal animal, - nebulization blank control group for model animal, - fudosteine tablet group (42.5 mg/kg) for model animal, - positive acetylcysteine solution for inhalation group for model animal, - high dose of fudosteine inhalation solution group (8.75 mg/kg) for model animal, - medium dose of fudosteine inhalation solution group (4.5mg/kg) for model animal, - low dose of fudosteine inhalation solution group (2.25 mg/kg) for model animal, with 10 rats in each group, half male and half female. All the animals in the model groups were inhaled with nebulized LPS solution (1 mg/mL, generated gas volume of 10 L/min, diluted gas volume of 1 L/min, 40 min for each time, once a day), and the models were established once a day for 2 consecutive days. Rats in each group were administered according to the dosage and route in Table 12, once a day for 3 consecutive days. Rats were anesthetized by intraperitoneal injection of chloral hydrate immediately after the last administration, and fixed in supine position. The neck skin was cut, the trachea was separated out, a glass capillary with a known weight was inserted between the two cartilages at the lower edge of the thyroid gland to absorb sputum, and the glass capillary was replaced with another one immediately after the sputum filled the capillary. The capillary weight gain was used as the detection index, and the tracheal secretion drainage fluid was collected, weighed and the 2h tracheal secretion volume was calculated (secretion volume = capillary weight gain/body weight * 100g).

After the drainage, the trachea from the throat to the bifurcation of the trachea was taken, weighed and cut open longitudinally, and airway mucus were removed by dipping with a cotton swab, washed with 3 mL distilled water and centrifuged at 3000 r/15min, and 2.5 mL supernatant was taken for later use. The content of mucopolysaccharide per unit weight of tracheal in rats in each group was determined by sulfuric acid-carbazole colorimetry at the wavelength of 530 nm.

In the process of acute and chronic airway diseases, the higher the secretion amount of mucus and mucin, the higher the glycosylation degree, which was closely related to the severity of infection. Mucin is a main component of mucus, which determines the adhesion, viscosity and elasticity of airway mucus. Glycosyls at the end of mucin are collectively called mucopolysaccharide, and its main components are sialic acid and fucose. Therefore, in the test, the mucopolysaccharide content was used as an index to evaluate the mucorepairing effect of the fudosteine inhalation solution. In addition, the amount of airway mucus secretion was measured simultaneously with the mucopolysaccharide content in the test.

In view of the characteristics that the test sample in this study was an inhalation preparation, a certain amount of inhaled solution will accumulate in the respiratory tract of animals after the animals are given with the test sample through the oral and nasal administration system, which will lead to the increase of secretion in the respiratory tract of animals, even if the blank control group and the model control group are given with saline control, the secretion fluid will still increase. Classical detection of airway secretion was carried out on normal animals, so it was difficult to objectively evaluate the effectiveness of inhalation preparations on airway secretion. Therefore, in this test, the normal control group under physiological condition and the mucus hypersecretion model control under pathological condition were designed to observe and compare the effects of the test samples on the secretion amount of animal mucus, and more importantly, to evaluate the effects of that on the content of mucopolysaccharide in airway mucus.

The results are shown in Table 12:

Table 12 Influence of airway secretion and mucopolysaccharide content in rats with mucus hypersecretion induced by LPS ($\bar{x}$, n=12)

| Groups | Dosage (mg/kg) | Secretion amount (mg/100g) | Content of mucopolysaccharide in airway ($\mu$g/mL/mg) |
|---|---|---|---|
| nebulization blank control group for normal animal | / | 5.11 | 15.92 |
| positive acetylcysteine solution for inhalation group for normal anima | 108 | 19.27 | 20.44 |
| high dose of fudosteine inhalation solution group for normal anima | 8.75 | 30.46 | 20.97 |
| medium dose of fudosteine inhalation solution group for normal anima | 4.5 | 21.83 | 20.48 |
| low dose of fudosteine inhalation solution group for normal anima | 2.25 | 10.63 | 21.13 |
| nebulization blank control group for model animal | / | 11.00 | 28.92 |
| fudosteine tablet group for model animal | 42.5 | 24.54 | 20.87 |
| positive acetylcysteine solution for inhalation group for model animal | 108 | 27.59 | 23.14 |
| high dose of fudosteine inhalation solution group for model animal | 8.75 | 26.09 | 19.42 |
| medium dose of fudosteine inhalation solution group for model animal | 4.5 | 24.25 | 18.36 |
| low dose of fudosteine inhalation solution group for model animal | 2.25 | 15.25 | 23.89 |

The results showed that, in the normal animal groups, compared with the nebulization blank control group, the secretion amounts in airway of rats in both high dose of fudosteine inhalation solution group and middle dose of fudosteine inhalation solution group increased significantly ($P < 0.01$; $P<0.05$), there was no significant difference in low dose group, but in model animal groups, the secretion amount in airway of rats in blank control group increased significantly ($P<0.01$), suggesting that the model was established successfully. In the model animal groups, compared with nebulization blank control group, the secretion amounts in airway of rats in fudosteine tablet group, positive acetylcysteine solution for inhalation group, high dose of fudosteine inhalation solution group and medium dose of fudosteine inhalation solution group increased significantly ($P < 0.01$; $P<0.05$), there was no significant difference in low dose group.

In the normal animal groups, compared with the nebulization blank control group, the contents of mucopolysaccharide in airway secretion of rats in positive acetylcysteine solution for inhalation group, high dose of fudosteine inhalation solution group, medium dose of fudosteine inhalation solution group and low dose of fudosteine inhalation solution group increased significantly ($P < 0.01$; $P<0.05$), but in model animal groups, the content of mucopolysaccharide in airway secretion of rats in nebulization blank control group increased significantly ($P<0.01$), suggesting that the model was established successfully. In the model animal groups, compared with nebulization blank control group, the contents of mucopolysaccharide in the airway secretion of rats in fudosteine tablet group, positive acetylcysteine solution for inhalation group, high dose of fudosteine inhalation solution group and medium dose of fudosteine inhalation solution group decreased significantly ($P < 0.01$; $P<0.05$), there was no significant difference in low dose group.

The test results showed that the mucus secretion amount in airway and mucopolysaccharide content of the test sample increased significantly after inhalation administration to normal rats. However, in the contrast test between normal model rats and mucus hypersecretion model rats, it could be observed the results that the test sample significantly reduced the mucopolysaccharide content. It showed that the test samples achieved the effect of inhibiting mucus hypersecretion by inhibiting the secretion of mucopolysaccharide in airway.

4.4. Influence on airway inflammation

SD species rats were randomly divided into 9 groups according to their body weight, that is, blank control group, model control group, positive prednisolone acetate group, high dose of fudosteine tablet group (42.5 mg/kg), medium dose of fudosteine tablet group (21 mg/kg), low dose of fudosteine tablet group (10 mg/kg), high dose of fudosteine inhalation solution group (8.75mg/kg), medium dose of fudosteine inhalation solution group (4.5 mg/kg) and low dose of fudosteine inhalation solution group (2.25 mg/kg), with 8 rats in each group, half male and half female. Except for the blank control group, animals in other groups were inhaled with nebulized LPS solution (1 mg/mL, generated gas volume 8 L/min, diluted gas volume 4 L/min, 40 min for each time and once a day), and the models were established once a day for 3 consecutive days. Animals in each group were administered according to the dosage and route in Table 13 one hour after modeling every day. Trachea and lung were taken out 4 hours after the last modeling, fixed in 10% formalin, dehydrated, embedded, sliced, and stained with HE, and the tissues were examined for pathology, the number of neutrophils in the tissues was counted, and the differences between groups were compared.

The results are shown in Table 13.

Table 13 Influence of airway inflammation in rats induced by LPS ($\bar{x}$, n=8)

| Groups | Dosage (mg/kg) | Number of neutrophil |
|---|---|---|
| blank control group | / | 0.2 |
| model control group | / | 10.5 |
| positive prednisolone acetate group | 12.6 | 7.8 |
| high dose of fudosteine tablet group | 42.5 | 7.4 |
| medium dose of fudosteine tablet group | 21 | 9.6 |
| low dose of fudosteine tablet group | 10 | 9.5 |
| high dose of fudosteine inhalation solution group | 8.75 | 5.6 |
| medium dose of fudosteine inhalation solution group | 4.5 | 7.9 |
| low dose of fudosteine inhalation solution group | 2.25 | 9.8 |

The results showed that, compared with the blank control group, inflammatory cells mainly composed of neutrophils were infiltrated in the airway of rats in the model control group, and the number of neutrophils increased significantly ($P < 0.01$). Compared with the model control group, the number of neutrophils in the positive prednisolone acetate group, high dose of fudosteine tablet group (42.5 mg/kg) and high dose of fudosteine inhalation solution group (8.75 mg/kg) and middle dose of fudosteine inhalation solution group (4.5 mg/kg) decreased significantly ($P < 0.01$; $P<0.05$). The number of neutrophils in medium dose of fudosteine tablet group, low dose of fudosteine tablet group, and low dose of fudosteine inhalation solution group was reduced, but there was no statistical difference.

Based on the research on the inhibition of goblet cell proliferation in the main bronchial epithelial tissue, and the inhibition of airway mucus secretion and airway inflammation, the above fudosteine inhalation solution as test sample were comprehensively evaluated for the expectorant effect. In the model of goblet cell proliferation in the main bronchial epithelial tissue of rats induced by LPS, a comparative study was made on the effective dose of fudosteine inhalation solution and control fudosteine tablets, and the $ED_{50}$ values of the two were calculated. The results showed that the minimum effective dose of fodotestan inhalation solution was 4.5 mg/kg, and the $ED_{50}$ value was 3.38 mg/kg. The minimum effective dose of fodotestan tablets was 42.5 mg/kg, and the $ED_{50}$ value was 37.67 mg/kg. The minimum effective doses of the two differed by 9.4 times, and the $ED_{50}$ values differed by 11.1 times.

**Application Example 7:** Investigation on the expectorant effect and its mechanism of fodotestan inhalation solution

Fudosteine oral solution and fudosteine inhalation solution were given to the LPS-induced phlegm model rats respectively, and their expectorant effects were compared.

1. Test materials

1.1. Test samples and control materials

Fudosteine inhalation solution (formula 1): colorless and clear liquid, 80 mg/mL, self-made.
Fudosteine tablet: 0.2 g/tablet, a white tablet, purchased from Jiangsu Zhengdafenghai Pharmaceutical Co., Ltd., with batch numbers of 2002151 and 2105141 respectively, and expiration dates of July, 2021 and April, 2023 respectively.

1.2. Reagent

Lipopolysaccharide (LPS): white crystal, 100 mg/bottle, purity ≥99.5%, with a batch number of 028M4094V, May, 2021, purchased from SIGMA Company.
Preparation method: a certain amount of LPS was accurately weighed and mixed with physiological saline to prepare an LPS solution with a concentration of 2 mg/mL.
Physiological saline: colorless and transparent liquid with a batch number of 1903111602.

1.3. Instruments

Analytical balance, CPA225D, produced by Sartorius Company.
Automatic film dyeing machine, DRS 2000JC-D2, SAKURA Company, Japan.
Microscope, BX43, OLYMPUS, Japan.

1.4. Animals

A total of 96 SD rats (half male and half female), weighing 200-220 g, were purchased from Beijing Weitong Lihua Experimental Animal Technology Co., Ltd. with the license number SCXK (Beijing) 2016-0006.

2. Test process

Rats were given with LPS solution by nebulizing inhalation to induce the proliferation of goblet cells in trachea and main bronchus, and phlegm models in rats were established, and the number of goblet cells in trachea and main bronchus of animals was measured, so as to evaluate the expectorant effect of the test samples.

Test method:

Rats passing quarantine inspection were randomly divided into blank group, model group, high dose of nebulization group, medium dose of nebulization group, low dose of nebulization group, high dose of oral group, medium dose of oral group and low dose of oral group, with 8 rats in each group (half male and half female). Except for the blank control group, the animals in other groups were administrated with LPS solution with a concentration of 2 mg/mL by nebulizing inhalation under the nebulization conditions of gas generation of 10 L/min and dilution of 1 L/min, 40 min for one time and once a day, twice, to establish a phlegm model of rat.
The blank group and the model group were given pure water by gavage the next day after the second modeling, and the other groups were given corresponding doses of test samples and control samples by nebulization inhalation or oral administration according to the dosage in Table 12, once a day for 5 consecutive days. The right lung was taken four hours after the last administration, and stained with AB-PAS. Three sections of lung bronchus were taken from each animal, and three random views were selected from each section for microscopic examination. The length of basement membrane and the number of goblet cells in each view were measured respectively, and the number of goblet cells contained on basement membrane per unit length (mm) was calculated. The average result in nine views was taken as the measurement result of goblet cells in the animal.

3. Test results

Compared with the blank group, the number of goblet cells in the lung bronchus of animals in the model group was increased significantly (*P* < 0.01). Compared with the model group, the number of goblet cells in the trachea and main bronchus of animals in the high dose of nebulization group, the middle dose of nebulization group and the low dose of nebulization group was decreased significantly (*P* < 0.01), but there was no significant change in the high dose of oral administration group, the middle dose of oral administration group and the low dose of oral administration group (*P* > 0.05), as shown in Table 14.

Table 14 Influence on goblet cells in trachea and main bronchus of rats in phlegm modal ($\bar{x}$, n=8)

| Groups | Dosage(mg/kg) | Number of goblet cell ( / mm) |
|---|---|---|
| blank group | - | 0.51 |
| model group | - | 10.05** |
| high dose of nebulization group | 8.75 | 3.60## |
| medium dose of nebulization group | 4.5 | 4.61## |
| low dose of nebulization group | 2.25 | 9.48 |
| high dose of oral group | 42.5 | 3.08## |
| medium dose of oral group | 21 | 6.35 |
| low dose of oral group | 10 | 7.94 |

The experimental results showed that compared with the blank control group, the number of goblet cells in the main bronchial epithelial tissue of rats on the model control group was increased significantly; compared with the model control group, the number of goblet cells in the main bronchial epithelial tissue of rats in the high dose of oral group, the high dose of nebulization group and the middle dose of nebulization group was significantly reduced, which had obvious expectorant effect (anti-goblet cell proliferation ability). The number of goblet cells in the epithelial tissue of the main bronchus of rats in the middle dose of oral group, the low dose of oral group and the low dose of nebulization group had a reduction trend, but there was no statistical difference. The minimum effective dose of the fudosteine inhalation solution in the phlegm model was 4.25 mg/kg, which was much lower than the 42.5 mg/kg of the control product (fudosteine tablets on the market). The nebulised inhalation solution composition and the assembly thereof in the present disclosure show good therapeutic effect.

The description of the above examples is only for helping to understand the method and core idea of the present disclosure. It should be pointed out that several improvements and modifications may be made on the present disclosure by those ordinary skilled in the art without departing from the principle of the present disclosure, and these improvements and modifications also fall within the protection scope of the claims of the present disclosure.

**Claims**

1. A fudosteine nebulised inhalation solution composition, comprising fudosteine or a pharmaceutically acceptable salt thereof as an active ingredient, a pH-adjusting agent and water for injection; wherein the composition has a pH of 2.5-5.0; and
the inhalation solution during nebulization inhalation has following droplet particle sizes: D90 particle size of 7.0-10.0 μm, D50 particle size of 2.8-4.5 μm, and D10 particle size of 0.5-1.5 μm; and the percent deposition of the particles with aerodynamic particle size of 1.4-5.4 μm in the fudosteine inhalation solution is not less than 50%.

2. The inhalation solution composition according to claim 1, wherein the percent deposition of the particles with aerodynamic particle size of 1.4-5.4 μm in the fudosteine inhalation solution is not less than 53%; and preferably, the percent deposition of the particles with aerodynamic particle size of 2.1-5.4 μm in the fudosteine inhalation solution is not less than 41.5%.

3. The inhalation solution composition according to claim 1, wherein the inhalation solution during nebulization inhalation has following droplet particle sizes: D90 particle size of 7.0-9.5 μm, D50 particle size of 2.8-4.4 μm, and D10 particle size of 0.5-1.0 μm; and preferably, the inhalation solution during nebulization inhalation has following droplet particle sizes: D90 particle size of 7.13-9.42 μm, D50 particle size of 2.81-4.38 μm, and D10 particle size of 0.54-0.95 μm.

4. The inhalation solution composition according to any one of claims 1 to 3, wherein the concentration of the active ingredient in the composition is 20-140 mg/ml.

5. The inhalation solution composition according to claim 4, wherein the concentration of the active ingredient in the composition is 80 mg/mL.

6. The inhalation solution composition according to any one of claims 1 to 3, wherein the composition has a pH of 3.5-4.0.

7. The inhalation solution composition according to claim 4, wherein the composition has a pH of 3.7-3.9.

8. The inhalation solution composition according to any one of claims 1 to 3, wherein the pH-adjusting agent is selected from the group consisting of hydrochloric acid, sulfuric acid, tartaric acid, lactic acid, citric acid, glacial acetic acid, malic acid, sodium citrate, sodium hydroxide, and a combination thereof.

9. An assembly comprising the fudosteine nebulised inhalation solution composition according to any one of claims 1 to 8,
comprising:

> (1) the nebulised inhalation solution composition according to any one of claims 1 to 8;
> (2) an nebulizing device for use in conjunction with the composition;

wherein, the nebulizing device comprises a nebulizer and a nebulizing cup; the nebulizer has a compressor pressure parameter of 1.1-1.6 bar and a spraying rate of 0.4-0.6 ml/min; the particles of 0.9 wt% standard sodium chloride solution excited by the nebulizing cup have an average particle diameter of 2.5-3.5 μm.

10. The assembly according to claim 9, wherein the nebulizer has a compressor pressure parameter of 1.2-1.6 bar and a spraying rate of 0.5-0.6 ml/min.

11. Use of the fudosteine nebulised inhalation solution composition according to any one of claims 1 to 8 or the assembly according to any one of claims 9 to 10 in the manufacture of a medicament product for expectorant treatment of a respiratory disease, comprising:

> (1) adding and dissolve fudosteine or pharmaceutically acceptable salt thereof as an active ingredient into water for injection;
> (2) adding a pH-adjusting agent into the solution of step (1) to adjust the pH of the solution to 3.7-3.9 to obtain an inhalation solution composition;
> (3) filtering, filling and sealing the inhalation solution composition; and
> (4) nebulizing the composition using a nebulizing device;
> wherein, the nebulizing device comprises a nebulizer and a nebulizing cup;
> the nebulizer has a compressor pressure parameter of 1.1-1.6 bar and a spraying rate of 0.4-0.6 ml/min, and the particles of the 0.9 wt% standard sodium chloride solution excited by the nebulizing cup have an average particle diameter of 2.5-3.5 μm.

12. The use according to claim 11, wherein the administration site of the medicament product includes one or more of nose, throat, trachea, esophagus and main bronchus.

13. The use according to claim 11, wherein the medicament product is used for expectorant treatment of one or more of bronchial asthma, chronic asthmatic bronchitis, bronchiectasis, tuberculosis, pneumoconiosis, chronic obstructive emphysema, atypical mycobacteriosis, pneumonia and diffuse bronchitis.

drug distribution results under different driver flow rates

FIG. 1

**EP 4 559 459 A1**

## INTERNATIONAL SEARCH REPORT

International application No.

**PCT/CN2023/107448**

### A. CLASSIFICATION OF SUBJECT MATTER

A61K9/72(2006.01)i; A61K9/08(2006.01)i; A61K31/198(2006.01)i; A61M11/00(2006.01)i; A61M15/00(2006.01)i; A61P11/00(2006.01)i; A61P11/06(2006.01)i; A61P11/10(2006.01)i; A61P31/06(2006.01)i

According to International Patent Classification (IPC) or to both national classification and IPC

### B. FIELDS SEARCHED

Minimum documentation searched (classification system followed by classification symbols)

A61K, A61M, A61P

Documentation searched other than minimum documentation to the extent that such documents are included in the fields searched

Electronic data base consulted during the international search (name of data base and, where practicable, search terms used)

CNABS, CNTXT, DWPI, WOTXT, EPTXT, USTXT, CNKI, PUBMED, ISI_Web of Science, Science Direct, STNext: 北京盈科瑞创新药物研究有限公司, 福多司坦, 雾化, 吸入, 雾滴, 粒径, D90, D50, D10, Fodosteine, Nebulization, Inhalation, Inhal+, Droplet, Particle Size

### C. DOCUMENTS CONSIDERED TO BE RELEVANT

| Category* | Citation of document, with indication, where appropriate, of the relevant passages | Relevant to claim No. |
|---|---|---|
| PX | CN 115068450 A (BEIJING INCREASEPHARM CO., LTD. et al.) 20 September 2022 (2022-09-20) abstract, and claims 1-13 | 1-13 |
| Y | WO 2019119720 A1 (BEIJING INCREASEPHARM CO., LTD.) 27 June 2019 (2019-06-27) claims 1-9, and description, prescription 7 | 1-13 |
| Y | CN 113768910 A (JOINCARE PHARMACEUTICAL GROUP INDUSTRY CO., LTD. et al.) 10 December 2021 (2021-12-10) claims 12-21, and description, paragraph 23 | 1-13 |
| A | CN 109745301 A (BEIJING INCREASEPHARM CO., LTD.) 14 May 2019 (2019-05-14) abstract, and claims 1-9 | 1-13 |
| A | CN 108078964 A (DISHA PHARMACEUTICAL GROUP CO., LTD. et al.) 29 May 2018 (2018-05-29) abstract, and claims 1-7 | 1-13 |

☑ Further documents are listed in the continuation of Box C.    ☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 August 2023** | **22 August 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

26

## INTERNATIONAL SEARCH REPORT

| | International application No. |
|---|---|
| | **PCT/CN2023/107448** |

| C. | DOCUMENTS CONSIDERED TO BE RELEVANT | |
|---|---|---|
| Category* | Citation of document, with indication, where appropriate, of the relevant passages | Relevant to claim No. |
| A | 陈荣昌主编 (CHEN, Rongchang, editor-in-chief). "雾化吸入装置 (Non-official translation: Atomization Inhalation Device)"<br>*呼吸与危重症医学2018-2019 (Non-official translation: Respiratory and Critical Disease Medicine 2018-2019)*, 31 August 2019 (2019-08-31), 673<br>August 2019, edition 1, and page 673 | 1-13 |

INTERNATIONAL SEARCH REPORT
Information on patent family members

International application No.

**PCT/CN2023/107448**

| Patent document cited in search report | | | Publication date (day/month/year) | Patent family member(s) | | | Publication date (day/month/year) |
|---|---|---|---|---|---|---|---|
| CN | 115068450 | A | 20 September 2022 | None | | | |
| WO | 2019119720 | A1 | 27 June 2019 | None | | | |
| CN | 113768910 | A | 10 December 2021 | None | | | |
| CN | 109745301 | A | 14 May 2019 | EP | 3708153 | A1 | 16 September 2020 |
| | | | | EP | 3708153 | A4 | 09 December 2020 |
| | | | | US | 2020261362 | A1 | 20 August 2020 |
| | | | | WO | 2019091082 | A1 | 16 May 2019 |
| | | | | JP | 2021502398 | A | 28 January 2021 |
| CN | 108078964 | A | 29 May 2018 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210842971
- CN 113975262 A
- CN 112057418 A
- CN 108078964 A
- US 20200316003 A1